# EUROPEAN PATENT APPLICATION

(11) **EP 4 311 538 A1**
(43) Date of publication of application: **31.01.2024**
(21) Application number: 22187088.4
(22) Date of filing: 26.07.2022
(51) Int. Cl.: A61K 8/99, A61Q 19/00, A61P 17/10, A61P 31/02, A61P 39/00

(54) **NOVEL SKIN CARE COMPOSITION FOR THE TREATMENT OF ACNE**

(71) Applicant: Beiersdorf AG, 20245 Hamburg (DE)
(72) Inventor: HÜPEDEN, Jennifer, 20251 Hamburg (DE); FÖLSTER, Heike, 22149 Hamburg (DE); REUTER, Jörn Hendrik, 24558 Henstedt-Ulzburg (DE); GALLINAT, Stefan, 22880 Wedel (DE); AHLE, Charlotte, 13591 Phöbener Steig 3c (DE)
(74) Representative: Uexküll & Stolberg

(57) **Abstract**

The present invention generally relates to the field of skin care. More particularly, the invention relates to a cosmetic or therapeutic skin care composition comprising at least one skin-health promoting *Staphylococcus* strain that exerts an antimicrobial activity against pathogenic *Cutibacterium acnes* strains, while not affecting non-pathogenic *Cutibacterium acnes* strains. The invention also provides a method for treating or preventing acne, oily skin, or dry skin by applying the skin care composition of the invention to a skin area in need of treatment. The invention also relates to the use of a skin care composition of the invention for treating or preventing acne, oily skin, or dry skin.

## Description

The present invention generally relates to the field of skin care. More particularly, the invention relates to a cosmetic or therapeutic skin care composition comprising at least one skin-health promoting *Staphylococcus* strain that exerts an antimicrobial activity against pathogenic *Cutibacterium acnes* strains, while not affecting non-pathogenic *Cutibacterium acnes* strains, or a culture supernatant of said *Staphylococcus* strain. The invention also provides a method for treating or preventing acne, oily skin, or dry skin by applying the skin care composition of the invention to a skin area in need of treatment. The invention also relates to the use of a skin care composition of the invention for treating or preventing acne, oily skin, or dry skin.

### BACKGROUND OF THE INVENTION

Common acne is a widespread long-term skin condition that affects more than 600 million people worldwide. Acne is most common among teenagers, though it affects people of all ages. It is normally caused by a combination of sebaceous gland hyperplasia, excessive sebum production, and impaired cornification. As a result, the hair follicles become plugged with oil and dead skin cells which leads to pimples and oily skin (Pschyrembel, Klinisches Wörterbuch, 258. ed., Walter de Gruyter-Verlag, Berlin, 1998). The colonization of the affected skin area with bacteria may additionally cause inflammation. Acne predominantly affects skin areas with a high number of sebaceous glands, in particular the face, the upper part of the chest, and the back. The occurrence of acne may lead to emotional distress and mental problems, such as reduced self-esteem and depression.

Oily skin is a transition state between healthy skin and acne-prone skin. In oily skin, the sebaceous glands of the skin produce an excessive amount of sebum which then serves as an ideal nutrient for a number bacteria and yeasts. These microorganisms decompose the sebum to glycerine and fatty acids, thereby further inducing the production of sebum in the sebaceous glands and destroying the follicle walls in the skin. This results in inflammation of the skin and the formation of pimples, pustules, nodules and cysts which often heal only with scarring which permanently affects the optical appearance of the subject's skin (W. Umbach [Ed.], Kosmetik, Entwicklung, Herstellung und Anwendung kosmetischer Mittel, 2. Edition Thieme Verlag, Stuttgart, 1995).

Today, the standard treatment for acne normally includes the topical application of antibiotics, including erythromycin, clindamycin, metronidazole, sulfacetamide, doxycycline or minocycline, to reduce the number of bacteria, especially C. acnes. However, some of these antibiotics exhibit considerable side effects which make their use inconvenient for the patient. Also, antibiotics normally act unspecifically and eradicate all bacteria located on the skin, including those which positively influence skin health. In addition, the treatment of acne with antibiotics is associated with high relapse rates due to the fact that small populations of pathogenic C. acnes survive and resume growth after termination of the antibiotic treatment. The anaerobic species *Cutibacterium acnes* (formerly *Propionibacterium acnes*) is thought to play an important role in the development of acne, since high numbers of these bacteria are regularly found in patients suffering from moderate or severe inflammatory acne. However, the underlying mechanisms are not completely understood. More recently, the putative relationship between acne and the human skin microbiome was analyzed (Belkaid and Segre, 2014; Oh et al., 2014). Specifically, it was suggested that acne might be the result of a distortion of the skin microbiome that is caused by specific strains of *C*. *acnes* (Holmes, 2013; Lomholt and Kilian, 2010). While the skin is colonized by a large number of microorganisms which are harmless or even beneficial (Grice and Segre, 2011), alterations of the microbiome can result in pathogenic conditions and skin diseases (Bek-Thomsen et al., 2008; Holmes, 2013; Kong et al., 2012, Fitz-Gibbon et al., 2013). This distortion may be caused by a specific subset of *C*. *acnes* strains (Lomholt and Kilian, 2010). On the other hand, it has been reported that another subset of *C*. *acnes* strains appears to be involved in stabilizing a healthy skin state.

Accordingly, there is a need for new methods of treating or preventing acne which specifically eradicate strains that induce or aggravate acne while preserving strains that positively influence skin health. The methods shall be essentially free of side effects and provide for a long-lasting effect.

### SUMMARY OF THE INVENTION

In a first aspect, the invention relates to a skin care composition for topical administration to the skin comprising one or more *Staphylococcus* strain that exerts a specific antimicrobial activity against pathogenic *C*. *acnes* strains or a culture supernatant obtained from such a *Staphylococcus* strain.

In the context of the present invention, a *Staphylococcus* strain is considered as exerting activity against a *C*. *acnes* strain, if the strain is active in the following lawn assay. A culture of the *Staphylococcus* strain is prepared and incubated overnight at 37°C in CASO broth until an OD₆₀₀ of 8.0 is reached. Subsequently, 15 µl of the overnight culture are pipetted to a lawn plate of the respective *C*. *acnes* strain which comprises at least 1×10⁸ CFU/plate. The *Staphylococcus* strain is active against the *C*. *acnes* strain, if a zone of inhibition appears around the site of application after anaerobic incubation of the plate for 96 hours at 37°C.

Preferably, the one or more *Staphylococcus* strain which exert the antimicrobial activity is selected from the group consisting of:
(a) *S. hominis* HAA254, deposited at the DSMZ under accession number DSM 34166,
(b) *S. hominis* HAA272, deposited at the DSMZ under accession number DSM 34167,
(c) *S. capitis* HAB56, deposited at the DSMZ under accession number DSM 34177,
(d) *S. capitis* HAB177, deposited at the DSMZ under accession number DSM 34178,
(e) *S. capitis* HAB198, deposited at the DSMZ under accession number DSM 34179,
(f) *S. capitis* HAB276, deposited at the DSMZ under accession number DSM 34197,
(g) *S. hominis* HAC286, deposited at the DSMZ under accession number DSM 34168,
*(h) S. capitis* HAC349, deposited at the DSMZ under accession number DSM 34198,
*(i) S. capitis* HAC470, deposited at the DSMZ under accession number DSM 34199,
*(j) S. capitis* HAC507, deposited at the DSMZ under accession number DSM 34200,
*(k) S. epidermidis* HAC588, deposited at the DSMZ under accession number DSM 34158,
*(l) S. epidermidis* HAF242, deposited at the DSMZ under accession number DSM 34159,
*(m) S. capitis* HAF401, deposited at the DSMZ under accession number DSM 34153,
*(n) S. epidermidis* HAF424, deposited at the DSMZ under accession number DSM 34160,
*(o) S. epidermidis* HAC26, deposited at the DSMZ under accession number DSM 34117.

Any of the above strains, alone or in combination, or a culture supernatant obtained from any of these strains, can be used in the composition of the present invention. In one embodiment, the *Staphylococcus* strain is present in the composition in lyophilized or spray-dried form. The *Staphylococcus* strain preferably is present in the composition in an amount of 10⁴-10¹¹ CFU/ml, preferably 10⁷-10¹⁰ CFU/ml. The *Staphylococcus* strain may be present in the composition in an amount of at least 0.5% (w/v) of the skin care composition.

In a particularly preferred embodiment, the skin care composition of the present invention comprises more than one of the above strains. When more than one of the *Staphylococcus* strains HAA254, HAA272, HAB56, HAB177, HAB198, HAB276, HAC286, HAC349, HAC470, HAC507, HAC588, HAF242, HAF401, HAF424 and HAC26 are used in combination in the same skin care composition, it is particularly preferred that these strains are present in the composition in approximately equal concentrations, such as 1:1 or 1:1:1.

If culture supernatant is used, said supernatant is preferably sterile-filtered before adding it to the skin care composition.

In one embodiment, the skin care composition further comprises one or more excipients, wherein said one or more excipients preferably comprise an emollient, a filler, a thickener, a solubilizer, an antioxidant, a preservative, a pH adjuster, a binder, a buffering agent, a colorant, a humectants, a exfoliating agent, a preservative, a plant extract, an essential oils, or a fragrance.

The skin care composition is preferably formulated as a gel, cream, ointment or lotion.

In a second aspect, the invention relates to a skin care composition for use in a method of treating acne, oily skin, and/or dry skin in a subject, for use in a method of reducing the number of pathogenic C. *acnes* cells on the skin of a subject, and/or for use in a method of treating an infection of the skin of a subject with pathogenic C. *acnes* cells.

In a third aspect, the invention relates to *S. hominis* HAA254, deposited at the DSMZ under accession number DSM 34166, *S. hominis* HAA272, deposited at the DSMZ under accession number DSM 34167, *S. capitis* HAB56, deposited at the DSMZ under accession number DSM 34177, *S. capitis* HAB177, deposited at the DSMZ under accession number DSM 34178, *S. capitis* HAB198, deposited at the DSMZ under accession number DSM 34179, *S*. *capitis* HAB276, deposited at the DSMZ under accession number DSM 34197, *S. hominis* HAC286, deposited at the DSMZ under accession number DSM 34168, *S. capitis* HAC349, deposited at the DSMZ under accession number DSM 34198, *S. capitis* HAC470, deposited at the DSMZ under accession number DSM 34199, *S. capitis* HAC507, deposited at the DSMZ under accession number DSM 34200, *S. epidermidis* HAC588, deposited at the DSMZ under accession number DSM 34158, *S. epidermidis* HAF242, deposited at the DSMZ under accession number DSM 34159, *S. capitis* HAF401, deposited at the DSMZ under accession number DSM 34153, *S. epidermidis* HAF424, deposited at the DSMZ under accession number DSM 34160, *S. epidermidis* HAC26, deposited at the DSMZ under accession number DSM 34117, or a culture supernatant obtained from any of these strains.

### DESCRIPTION OF THE FIGURE

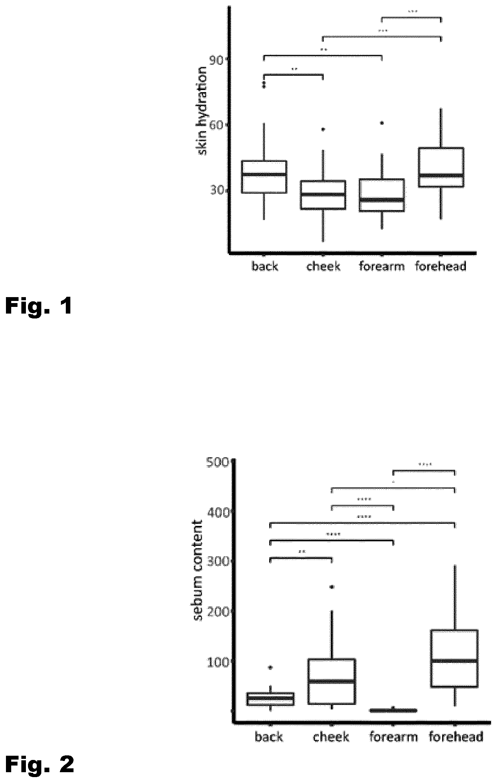

Figure 1 shows the results from the skin hydration measurement at the locations from which coagulase-negative staphylococci were isolated. Skin hydration data are shown for back, cheek, forearm, and forehead skin of the test subjects (^{∗}p ≤ 0.05, ^{∗∗}p ≤ 0.01, ^{∗∗∗}p ≤ 0.001, ^{∗∗∗∗}p ≤ 0.0001, unpaired Wilcoxon test).
Figure 2 shows the results from the sebum content measurement at the locations from which coagulase-negative staphylococci were isolated. Sebum content data are shown for back, cheek, forearm, and forehead skin of the test subjects (^{∗}p ≤ 0.05, ^{∗∗}p ≤ 0.01, ^{∗∗∗}p ≤ 0.001, ^{∗∗∗∗}p ≤ 0.0001, unpaired Wilcoxon test).

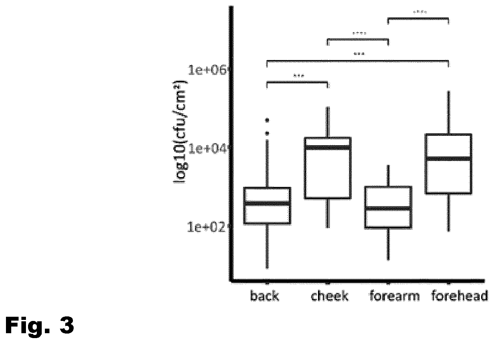

Figure 3 shows the results from the cell count measurement at the locations from which coagulase-negative staphylococci were isolated. Cell count results in colony-forming units (CFU) per cm² skin surface are shown for back, cheek, forearm, and forehead skin of the test subjects (^{∗}p ≤ 0.05, ^{∗∗}p ≤ 0.01, ^{∗∗∗}p ≤ 0.001, ^{∗∗∗∗}p ≤ 0.0001, unpaired Wilcoxon test).

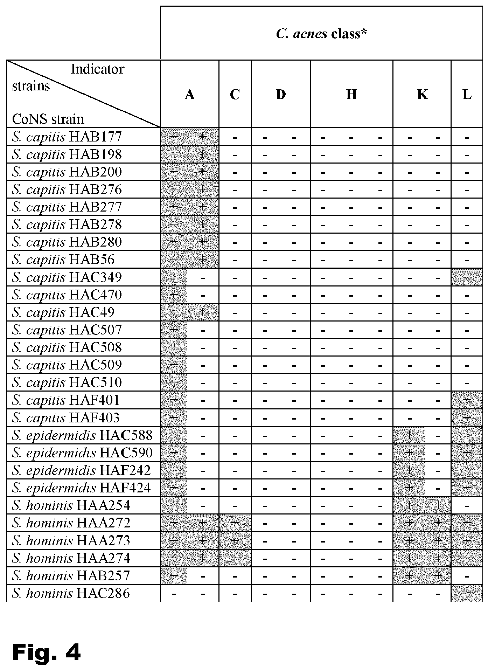

Figure 4 shows the antimicrobial activity of the *Staphylococcus* strains of the invention against C. *acnes* strains from different SLST classes.

### DETAILED DESCRIPTION

The present invention provides novel *Staphylococcus* strains that were isolated from natural environments and show a significant activity against pathogenic *C*. *acnes* strains, and in particular *C*. *acnes* strains that occur on skin that is afflicted with acne. When applied to the skin, these strains effectively reduce the number pathogenic *C*. *acnes* cells on the skin by secreting antibacterial compounds into the surrounding medium that selectively kill pathogenic *C*. *acnes* cells. The invention also relates to culture supernatants of any of theses strains.

*C. acnes* is a polyphyletic species that can be divided into different subspecies and phylotypes, namely, IA₁, IA₂, IB, IC, II and III (Lomholt et al. 2010; McDowell et al. 2013). To enable characterization of mixed populations of *C*. *acnes,* a single locus sequence typing (SLST) scheme has been developed that enables the differentiation into ten classes (A to L) (Scholz et al. 2014). SLST classes A to E correspond to phylotype IA₁ strains, whereas SLST classes F, G, H, K and L correspond to phylotypes IA₂, IC, IB, II and III, respectively (Scholz et al. 2014). Recent work has shown that some phylotypes/SLST classes are enriched in individuals with the skin disorder acne vulgaris, whereas others have been identified as markers of healthy skin. Acne-associated phylotypes include SLST classes A and C (both phylotype IA₁) and F (IA₂), whereas healthy skin is colonized with more diverse populations with a higher prevalence of strains belonging to the SLST classes H (IB) and K (II) (Dagnelie et al. 2018; Lomholt et al. 2017; McDowell et al. 2012; McDowell et al. 2011; Nakase et al. 2017; Nakase et al. 2020).

The *Staphylococcus* strains identified in the course of present invention have been found to be significantly more active against *C*. *acnes* strains which are associated with skin suffering from acne, in particular strains of the SLST classes A, C and F. At the same time, these strains show reduced or no activity against *C*. *acnes* strains which are associated with healthy skin, in particular strains of the SLST classes H and K.

In one preferred embodiment, the invention provides a *Staphylococcus* strains that is active against one or more *C*. *acnes* strains of SLST class A. In another embodiment, the invention provides a *Staphylococcus* strains that is active against one or more *C*. *acnes* strains of SLST classes A and C. In yet another embodiment, the invention provides a *Staphylococcus* strains that is active against one or more *C*. *acnes* strains of SLST classes A, C and F. In one preferred embodiment, the invention provides a *Staphylococcus* strains that is active against one or more *C*. *acnes* strains of SLST class A, but is not active against *C*. *acnes* strains of SLST class H. In another embodiment, the invention provides a *Staphylococcus* strains that is active against one or more *C*. *acnes* strains of SLST classes A and C, but is not active against *C. acnes* strains of SLST class H. In yet another embodiment, the invention provides a *Staphylococcus* strains that is active against one or more *C*. *acnes* strains of SLST class A, but is not active against *C*. *acnes* strains of SLST classes H and K. In yet another embodiment, the invention provides a *Staphylococcus* strains that is active against one or more *C*. *acnes* strains of SLST classes A and C, but is not active against *C*. *acnes* strains of SLST classes H and K.

The present invention therefore also provides a skin care composition for topical administration to the skin comprising one or more *Staphylococcus* strains that exert a specific antimicrobial activity against pathogenic *C*. *acnes* strains. The invention also relates to culture supernatants of any of theses strains. The one or more *Staphylococcus* strain is preferably selected from the group consisting of:
(a) *S. hominis* HAA254, deposited at the DSMZ under accession number DSM 34166,
(b) *S. hominis* HAA272, deposited at the DSMZ under accession number DSM 34167,
(c) *S. capitis* HAB56, deposited at the DSMZ under accession number DSM 34177,
(d) *S. capitis* HAB177, deposited at the DSMZ under accession number DSM 34178,
(e) *S. capitis* HAB198, deposited at the DSMZ under accession number DSM 34179,
(f) *S. capitis* HAB276, deposited at the DSMZ under accession number DSM 34197,
(g) *S. hominis* HAC286, deposited at the DSMZ under accession number DSM 34168,
*(h) S. capitis* HAC349, deposited at the DSMZ under accession number DSM 34198,
*(i) S. capitis* HAC470, deposited at the DSMZ under accession number DSM 34199,
*(j) S. capitis* HAC507, deposited at the DSMZ under accession number DSM 34200,
*(k) S. epidermidis* HAC588, deposited at the DSMZ under accession number DSM 34158,
*(l) S. epidermidis* HAF242, deposited at the DSMZ under accession number DSM 34159,
*(m) S. capitis* HAF401, deposited at the DSMZ under accession number DSM 34153,
*(n) S. epidermidis* HAF424, deposited at the DSMZ under accession number DSM 34160,
*(o) S. epidermidis* HAC26, deposited at the DSMZ under accession number DSM 34117.

Owing to their ability to control and reduce the abundance of pathogenic C. *acnes* strains on human skin, these strains or culture supernatants obtained from these strains can be used as active ingredients in cosmetic and pharmaceutical skin care compositions which are topically applied to skin comprising an excessive number of one or more pathogenic C. *aces* strains. Without wishing to be bound by theory, it is assumed that the activity of the strains of the invention is based on the production and secretion of antibacterial compounds that are active against pathogenic *S. aureus* strains. As these antibacterial compounds are secreted into the surrounding medium, it is also possible to use culture supernatants which are obtained from said strain. As used herein, culture supernatants are fractions of the cell culture medium that had been used for culturing the cells and in which compounds that were secreted from the cultured cells were accumulated.

Preferably, the one or more *Staphylococcus* strain is present in the skin care composition of the invention in lyophilized or spray-dried form. This means that viable bacteria have been subjected to a drying process that maintains their viability, but reduces their metabolic processes to minimum. In lyophilized or spray-dried form, the bacteria can be stored for months or even years. Once they are applied to the skin, such as the human skin, the metabolism of the bacteria is reactivated such that they resume growth. They propagate on the skin surface and displace pathogenic bacterial strains, thereby recovering a diverse, healthy and balanced skin microbiome.

In one embodiment, the one or more *Staphylococcus* strains are present in spray-dried form. The principle of spray drying is based on the dispersion of a solution into fine droplets which are introduced into a flow of hot air. The solvent evaporates from the substrate droplets so that dry product clusters remain. Standard spray drying devices can be used, such as the Mini Spray Dryer B-290 from Büchi Labortechnik GmbH (Essen, Germany) or the Mobile Minor^{™} Spray Dryer from GEA (Berlin, Germany). In another embodiment, one or more *Staphylococcus* strains are present in freeze-dried or lyophilized form. Freeze drying or lyophilization is a process which includes freezing the product, reducing the pressure and adding heat to allow the frozen water in the material to sublimate. Various methods can be applied for freezing the product. For example, freezing can be achieved by using a standard freezer or a chilled bath. Cooling the product below its triple point ensures that sublimation will occur upon heating. To prevent the formation of large crystals that may damage the structure of the product to be dried, freezing is done rapidly. About 95% of the water in the product is removed when the frozen water sublimates. Most materials can be dried to 1-5% residual moisture. Standard freeze drying devices can be used, such as the Lyovac^{™} devices from GEA (Berlin, Germany), the Gamma 2-20 Freeze dryer LCM-1 from Christ (Osterode am Harz, Germany), or the Christ Martin^{™} Alpha 1-2 Lyophilisator from Fisher Scientific GmbH (Schwerte, Germany).

It is preferred that the *Staphylococcus* strain is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *Staphylococcus* strain HAA254, strain HAA272, strain HAB56, strain HAB177, strain HAB198, strain HAB276, strain HAC286, strain HAC349, strain HAC470, strain HAC507, strain HAC588, strain HAF242, strain HAF401 or strain HAF424 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *Staphylococcus* strain is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In one embodiment *S. hominis* strain HAA254 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. hominis* strain HAA254 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. hominis* strain HAA254 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In another embodiment *S. hominis* strain HAA272 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. hominis* strain HAA272 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. hominis* strain HAA272 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In yet another embodiment *S. capitis* strain HAB56 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. capitis* strain HAB56 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. capitis* strain HAB56 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In yet another embodiment *S. capitis* strain HAB177 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. capitis* strain HAB177 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. capitis* strain HAB177 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In yet another embodiment *S. capitis* strain HAB198 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. capitis* strain HAB198 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. capitis* strain HAB198 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In yet another embodiment *S. capitis* strain HAB276 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. capitis* strain HAB276 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. capitis* strain HAB276 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In yet another embodiment *S. hominis* strain HAC286 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. hominis* strain HAC286 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. hominis* strain HAC286 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In yet another embodiment *S. capitis* strain HAC349 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. capitis* strain HAC349 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. capitis* strain HAC349 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In yet another embodiment *S. capitis* strain HAC470 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. capitis* strain HAC470 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. capitis* strain HAC470 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In yet another embodiment *S. capitis* strain HAC507 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. capitis* strain HAC507 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. capitis* strain HAC507 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In yet another embodiment *S. epidermidis* strain HAC588 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. epidermidis* strain HAC588 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. epidermidis* strain HAC588 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In yet another embodiment *S. epidermidis* strain HAF242 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. epidermidis* strain HAF242 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. epidermidis* strain HAF242 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In yet another embodiment *S. capitis* strain HAF401 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. capitis* strain HAF401 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. capitis* strain HAF401 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In yet another embodiment *S. epidermidis* strain HAF424 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. epidermidis* strain HAF424 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. epidermidis* strain HAF424 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In yet another embodiment *S. epidermidis* strain HAC26 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. epidermidis* strain HAC26may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. epidermidis* strain HAC26 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

Stated differently, the skin care composition of the invention comprises an amount of at least 0.5% (w/v) of the *Staphylococcus* strain, based on the overall volume of the skin care composition. For example, the skin care composition of the invention comprises an amount of at least 0.5% (w/v) of the *Staphylococcus* strain HAA254, strain HAA272, strain HAB56, strain HAB177, strain HAB198, strain HAB276, strain HAC286, strain HAC349, strain HAC470, strain HAC507, strain HAC588, strain HAF242, strain HAF401 or strain HAF424.

More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S*. *epidermidis* strain, based on the overall volume of the skin care composition.

In one embodiment, the skin care composition of the invention comprises an amount of at least 0.5% (w/v) of the *S. hominis* strain HAA254, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. hominis* strain HAA254, based on the overall volume of the skin care composition.

In another embodiment, the skin care composition of the invention comprises an amount of at least 0.5% (w/v) of the *S. hominis* strain HAA272, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. hominis* strain HAA272, based on the overall volume of the skin care composition.

In yet another embodiment, the skin care composition of the invention comprises an amount of at least 0.5% (w/v) of the *S. capitis* strain HAB56, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. capitis* strain HAB56, based on the overall volume of the skin care composition.

In yet another embodiment, the skin care composition of the invention comprises an amount of at least 0.5% (w/v) of the *S. capitis* strain HAB177, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. capitis* strain HAB177, based on the overall volume of the skin care composition.

In yet another embodiment, the skin care composition of the invention comprises an amount of at least 0.5% (w/v) of the *S. capitis* strain HAB198, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. capitis* strain HAB198, based on the overall volume of the skin care composition.

In yet another embodiment, the skin care composition of the invention comprises an amount of at least 0.5% (w/v) of the *S. epidermidis* strain HAB276, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. epidermidis* strain HAB276, based on the overall volume of the skin care composition.

In yet another embodiment, the skin care composition of the invention comprises an amount of at least 0.5% (w/v) of the *S. hominis* strain HAC286, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. hominis* strain HAC286, based on the overall volume of the skin care composition.

In yet another embodiment, the skin care composition of the invention comprises an amount of at least 0.5% (w/v) of the *S. capitis* strain HAC349, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. capitis* strain HAC349, based on the overall volume of the skin care composition.

In yet another embodiment, the skin care composition of the invention comprises an amount of at least 0.5% (w/v) of the *S. capitis* strain HAC470, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. capitis* strain HAC470, based on the overall volume of the skin care composition.

In yet another embodiment, the skin care composition of the invention comprises an amount of at least 0.5% (w/v) of the *S. capitis* strain HAC507, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. capitis* strain HAC507, based on the overall volume of the skin care composition.

In yet another embodiment, the skin care composition of the invention comprises an amount of at least 0.5% (w/v) of the *S. epidermidis* strain HAC588, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. epidermidis* strain HAC588, based on the overall volume of the skin care composition.

In yet another embodiment, the skin care composition of the invention comprises an amount of at least 0.5% (w/v) of the *S. epidermidis* strain HAF242, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. epidermidis* strain HAF242, based on the overall volume of the skin care composition.

In yet another embodiment, the skin care composition of the invention comprises an amount of at least 0.5% (w/v) of the *S. capitis* strain HAF401, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. capitis* strain HAF401, based on the overall volume of the skin care composition.

In yet another embodiment, the skin care composition of the invention comprises an amount of at least 0.5% (w/v) of the *S. epidermidis* strain HAF424, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. epidermidis* strain HAF424, based on the overall volume of the skin care composition.

In yet another embodiment, the skin care composition of the invention comprises an amount of at least 0.5% (w/v) of the *S. epidermidis* strain HAC26, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. epidermidis* strain HAC26, based on the overall volume of the skin care composition.

When more than one of the *Staphylococcus* strains are used in combination in the same skin care composition, each of the strains is preferably present in the composition in an amount of 1.0 × 10⁴-1.0 × 10¹¹ CFU/ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, each of the strains may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that each of the strains is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In some embodiments, the overall amount of lyophilized or spray-dried bacteria in the composition is 1.0 × 10⁴-1.0 × 10¹¹ CFU/ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the bacteria may be collectively present in the composition in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the bacteria are collectively present in the composition in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition. One of ordinary skill in the art will be readily able to determine the amount of bacteria in a lyophilized or spray-dried composition.

When more than one of the *Staphylococcus* strains HAA254, HAA272, HAB56, HAB177, HAB198, HAB276, HAC286, HAC349, HAC470, HAC507, HAC588, HAF242, HAF401, HAF424 and HAC26 are used in combination in the same skin care composition, it is particularly preferred that these strains are present in the composition in approximately equal concentrations, such as 1:1 or 1:1:1.

In one embodiment, the culture supernatant of a culture of one or more of the strains of the invention is added to the skin care composition of the invention. Preferably, the culture supernatant is sterile-filtered before adding it to the skin care composition. For example, the culture supernatant can be sterile-filtered by centrifugation of an overnight culture for 10 min at 8000 g followed by filtration of the supernatant with a filter having a pore size of 0.2 µm.

The skin care composition of the invention can be formulated as a liquid, ointment, cream, scrub, lotion, paste, gel, hydrogel, foam, or powder. It is however preferred that is formulated as a gel, cream, ointment or lotion. The skin care composition may be formulated be packaged into a pump dispenser which allows spraying the liquid onto the skin areas to be treated. The skin care composition of the invention can also be incorporated into a patch which is applied to the skin.

The composition of the invention may optionally comprise one or more excipients that are commonly used in cosmetic or pharmaceutical skin care products. Such excipients are described in detail, for example, in the International Cosmetic Ingredient Dictionary and Handbook, 16th ed. (2016). For example, the one or more excipients may comprise an emollient, an exfoliating agent, a humectant, a pH adjuster, a thickener, a solubilizer, an antioxidant, a preservative, a filler, a binder, a buffering agent, a colorant, an essential oil, a pigment, a sunscreen, an antiseptic, a chelating agent, a vitamin, panthenol, ubiquinone Q10, hyaluronic acid, or any combinations thereof.

For example, the skin care composition of the invention may comprise an emollient. As used herein, an emollient is a compound that moisturizes and/or softens the skin. Emollients normally reduce the roughness, cracking and/or irritation of the skin by penetrating into the deeper layers of the skin. Suitable emollients for the skin care composition of the present invention include, but are not limited to, olive oil, palm oil, soybean oil, sesame seed oil, rapeseed oil, evening primrose oil, sunflower seed oil, avocado oil, olive oil, coconut oil, castor oil, safflower seed oil, myristyl lactate, isopropyl myristate, polyethylene glycol, isopropyl palmitate, isopropyl stearate, isobutyl palmitate, isocetyl stearate, or cetyl alcohol. Preferably, the emollient is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the emollient is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one emollient. In this case the above amounts preferably refer to the overall amount of emollients in the composition.

The skin care composition of the invention may also comprise an exfoliating compound. Exfoliating compounds are solids that provide for a granular texture of the composition. When rubbed onto the skin, these compounds eliminate old skin cells and enhance skin renewal. Exfoliating compounds which are useful in the compositions of the present invention include, but are not limited to, urea, alpha-hydroxy acids and beta-hydroxy acids, and their esters, anhydrides, and salts. Suitable hydroxy acids include, for example, urea, glycolic acid, lactic acid, malic acid, mandelic acid, tartaric acid, citric acid, 2-hydroxyalkanoic acid, salicylic acid, and derivatives thereof. The use of urea is particularly preferred, because it also has an additional water-binding effect and enhances the absorption of other cosmetic ingredients. Preferably, the exfoliating compound is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the exfoliating compound is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one exfoliating compound. In this case the above amounts preferably refer to the overall amount of exfoliating compounds in the composition.

The skin care composition of the present invention may also comprise a humectant for improving skin hydration. Suitable humectants for use in the composition of the present invention include, but are not limited to, glycerine, polyethylene glycol ethers of glycerine, amino acids, such as proline and arginine, sugar and sugar alcohols, such as glucose, mannose, trehalose, and polyglycerol sorbitol, 1,3-butylene glycol, propylene glycol, diglycerol, glycerol monopropoxylate, glycogen, sodium hyaluronate, sodium poly-aspartate, sodium polyglutamate, sorbeth 20, sorbeth 6, and hydrogenated starch hydrolysates. Preferably, the humectant is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the humectant is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one humectant. In this case the above amounts preferably refer to the overall amount of humectants in the composition.

In one preferred embodiment, the skin care composition of the present invention comprises a compound that serves as a pH adjuster. Since the composition of the invention is used on the human skin, it will normally have a slightly acidic pH to make it more compatible with the acidic environment of the skin. The composition may have a pH in the range from about 2.5 to about 6.5, preferably from about 4.0 to about 6.0, and more preferably from about 5.0 to about 6.0 or from about 5.5 to about 6.0. The acidic pH can be achieved by adding an acid to the skin care composition of the invention, e.g. a carboxylic acid, such as an alpha hydroxy acid. The nature of the acid that can be used in the composition of the invention is not particularly limited. Suitable acids include formic acid, acetic acid, propionic acid, butyric acid, valeric acid, caproic acid, enanthic acid, caprylic acid, and the like. In a particular preferred embodiment, the composition comprises lactic acid as a pH adjuster. For application to the human skin, lactic acid is particularly useful, as it is also secreted by the skin flora to form the protective acidic milieu on the human skin surface. Preferably, the pH adjuster is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the pH adjuster is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one pH adjuster. In this case the above amounts preferably refer to the overall amount of pH adjusters in the composition.

In one preferred embodiment, the skin care composition of the present invention comprises a thickener. Thickeners are compounds that increase the viscosity of a cosmetic or pharmaceutical formulation. Thickeners are often polymers that absorb water and swell up, thereby making the composition more viscous. Suitable thickeners for the skin care composition of the present invention include, but are not limited to, bean gum, xanthan gum, gelatin, Carnauba wax, stearic acid, C. crispus extract, hydroxypropyl starch phosphate, and mixtures thereof. Preferably, the thickener is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the thickener is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one thickener. In this case the above amounts preferably refer to the overall amount of thickeners in the composition.

In one preferred embodiment, the skin care composition of the present invention comprises a solubilizer. As used herein, a solubilizer is a compound that aids in the solubilization of hydrophobic substances in aqueous and alcoholic formulations. For example, a solubilizer may render feasible the solubilization of perfume oils and other hydrophobic substances, such as vitamins, into an aqueous skin care composition. Suitable solubilizers for the skin care composition of the present invention include, but are not limited to, pentaerythrityl tetraisostearate, polyglyceryl-4 caprate, polyglyceryl-3 cocoate, polyglyceryl-10 caprylate and different poloxamers, such as poloxamer 188, 234, 235, 238 and 338. Preferably, the solubilizer is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the solubilizer is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one solubilizer. In this case the above amounts preferably refer to the overall amount of solubilizers in the composition.

In one preferred embodiment, the skin care composition of the present invention comprises an antioxidant. These compounds are normally added to cosmetic or pharmaceutical formulations to prevent oxidative reactions catalyzed by oxygen radicals that would otherwise result in the decomposition of ingredients in the composition, such as proteins, sugars, and lipids. Antioxidants which are commonly used in cosmetic product include chemicals like butylated hydroxytoluene, butylated hydroxyanisole, tocopherol, tocopheryl acetate and plant-derived polyphenols, flavonoids, flavanols, stilbens, and terpenes. Preferably, the antioxidant is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the antioxidant is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one antioxidant. In this case the above amounts preferably refer to the overall amount of antioxidants in the composition.

In one preferred embodiment, the skin care composition of the present invention comprises a preservative. As used herein, a preservative is a compound that is added to cosmetic or pharmaceutical formulation to prevent microbial spoilage of the formulation by inhibiting the growth of unintended bacteria and yeasts. Commonly used preservatives for cosmetic formulations include, amongst others, benzyl alcohol, ethanol, phenoxyethanol, caprylyl glycol, salicylic acid and sorbic acid. Preferably, the preservative is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the preservative is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one preservative. In this case the above amounts preferably refer to the overall amount of preservatives in the composition.

As used herein, all percentages are by weight of the skin care composition, unless specifically stated otherwise. All ratios are weight ratios, unless stated otherwise.

The novel strains of the present invention as well as culture supernatants obtained from any of these strains and skin care compositions comprising one or more of the recited strains or culture supernatants thereof are useful for the modulation of the skin microbiome, and in particular for reducing the number of pathogenic C. *acnes* cells on the skin of a subject. The strains, culture supernatants and skin care compositions of the invention are therefore particularly useful for treating skin infections with pathogenic *C*. *acnes* and skin diseases which characterized by an excessive amount of pathogenic *C*. *acnes* cells on the skin, such as oily skin and/or acne.

Thus, in one preferred aspect, the invention relates to one of the above recited *Staphylococcus* strains or a culture supernatant obtained from such a strain or a combination of two or more of these strains or culture supernatants thereof or a skin care composition described hereinabove comprising one or more of these strains or culture supernatants thereof for use in a method of reducing the number of pathogenic *C*. *acnes* cells on the skin of a subject. The invention also relates to the use of one of the above recited *Staphylococcus* strain or a culture supernatant obtained from such a strain or a combination of two or more of these strains or culture supernatants thereof or a skin care composition as described hereinabove comprising one or more of these strains or culture supernatants thereof for reducing the number of pathogenic *C*. *acnes* cells on the skin of a subject. The invention also relates to a method of reducing the number of pathogenic *C*. *acnes* cells on the skin of a subject, said method comprising the administration of one of the above recited *Staphylococcus* strains or a culture supernatant thereof or a combination of these strains or culture supernatants thereof or a skin care composition as described hereinabove comprising one or more of these strains or culture supernatants thereof to the skin of a subject in need thereof. It is particularly preferred that the subject to be treated is a human.

In another preferred aspect, the invention relates to one of the above recited *Staphylococcus* strains or a culture supernatant obtained from such a strain or a combination of these strains or culture supernatants thereof or a skin care composition described hereinabove comprising one or more of these strains or culture supernatants thereof for use in a method of treating a infection of the skin with pathogenic *C*. *acnes* in a subject. The invention also relates to the use of one of the above recited *Staphylococcus* strains or a culture supernatant obtained from such a strain or a combination of these strains or culture supernatants thereof or a skin care composition as described hereinabove comprising one or more of these strains or culture supernatants thereof for treating a infection of the skin with pathogenic *C*. *acnes* in a subject. The invention also relates to a method of treating a infection of the skin with pathogenic *C*. *acnes* in a subject, said method comprising the administration of the above recited *Staphylococcus* strains or a culture supernatant obtained from such a strain or a combination of these strains or culture supernatants thereof or a skin care composition as described hereinabove comprising one or more of these strains or culture supernatants thereof to the skin of a subject in need thereof. It is particularly preferred that the subject to be treated is a human.

In yet another preferred aspect, the invention relates to one of the above recited *Staphylococcus* strains or a culture supernatant obtained from such a strain or a combination of these strains or culture supernatants thereof or a skin care composition described hereinabove comprising one or more of these strains or culture supernatants thereof for use in a method of treating oily skin, acne and/or dry skin in a subject. The invention also relates to the use of one of the above recited *Staphylococcus* strains or a culture supernatant obtained from such a strain or a combination of these strains or culture supernatants thereof or a skin care composition as described hereinabove comprising one or more of these strains or culture supernatants thereof for treating oily skin, acne and/or dry skin in a subject. The invention also relates to a method of treating oily skin, acne and/or dry skin in a subject, said method comprising the administration of one of the above recited *Staphylococcus* strains or a culture supernatant obtained from such a strain or a combination of these strains or culture supernatants thereof or a skin care composition as described hereinabove comprising one or more of these strains or culture supernatants thereof to the skin of a subject in need thereof. It is particularly preferred that the subject to be treated is a human.

The skin to be treated may include the skin of the face and the body, e.g. the skin of the neck, chest, back, arms, hands, legs or thighs. According to a preferred embodiment, the skin to be treated with the composition of the present invention is the skin of the face. According to another preferred embodiment, the skin to be treated with the composition of the present invention is the skin of the body.

The skin care compositions may be applied to the area of skin in need of treatment, e.g. the face or body, at least once a day, twice a day, or even more frequently if needed. When applied twice daily, the first and second applications are preferably separated by at least 6 hours, preferably 8 hours. Typically, the cosmetic composition is applied once in the morning and once in the evening. The composition of the invention can be used over long periods without any adverse side effects. For example, the period of treatment may be at least 1 week, at least 2 weeks, at least 3 weeks, at least 4 weeks, at least 6 weeks, at least 12 weeks, at least 24 weeks, or more. In some embodiments, the treatment will be extended for several months, such as for 4 months, 6 months, 8 months, 12 months, 18 months, or 24 months.

The skin care composition of the present invention may be provided as ready-to-use composition which allows for a direct topical administration to the skin. In such a composition, the lyophilized or spray-dried live bacteria will be present in admixture with other cosmetic or pharmaceutical excipients described elsewhere herein, such as emollients, fillers, and the like. Upon application of these compositions to the skin, the dried bacteria will be re-activated on the skin of the subject to which the product is applied. Growth of the reactivated bacteria from the skin care composition will positively influence the microbial flora on the skin of the subject.

These ready-to-use compositions are preferably stable at room temperature for at least 1 week, at least 2, weeks, at least 3 weeks, at least 4 weeks, at least 5 weeks, at least 6 weeks, at at least 8 weeks, at least 10 weeks, at least 12 weeks, at least 14 weeks, at least 16 weeks, at least 18 weeks, at least 20 weeks, at least 22 weeks, at least 24 weeks, at least 26 weeks, at least 28 weeks, or at least 30 weeks or more. As used herein, a composition is regarded as being stable if the reduction in the number of colony forming units present in the composition after storage is less than a 3 log reduction, preferably less than a 2 log reduction, and more preferably less than a 1 log reduction. Stated differently, a composition is regarded as being stable if the reduction in the number of colony forming units present in the composition after storage is less than 1000-fold, preferably less than 100-fold, and more preferably less 10-fold relative to the number of colony forming units in the composition before storage.

The skin care compositions of the present invention may alternatively be provided as a kit-of-parts in which the lyophilized or spray-dried bacteria are spatially separated from the other components, e.g. the cosmetic or therapeutic components. For example, the kit-of-parts may be in the form of a packaging with two spatially separated chambers, wherein the first chamber contains the lyophilized or spray-dried bacteria, and the second chamber contains a cosmetic preparation, such as a water-containing cosmetic preparation. Prior to use, the contents of both chambers are mixed with each other, such as for example by a consumer or a patient, to provide a homogeneous skin care composition which is then applied to the skin. A kit-of-part assembly has the advantage that the bacteria can remain in lyophilized or spray-dried form until use which is associated with a particular high storage stability of the composition. In a kit-of-parts assembly, it is advantageous if the weight ratio of the bacteria in the first chamber to the cosmetic preparation, in particular the water-containing cosmetic preparation, in the second chamber is from 1:10 to 1:100, such as 1:10, 1:20, 1:30, 1:40, 1:50, 1:60, 1:70, 1:80, 1:90, or 1:100. After mixing the contents of both chambers, the skin care composition contains preferably 1-10% by weight lyophilized or spray-dried bacteria and 99-90% by weight of the cosmetic preparation, e.g. the water-containing cosmetic preparation. According to the present invention a kit-of-parts can be provided, for example, in a Lyo-Ject^{®} double-chamber syringe, in a V-LK^{®} double-chamber carpuel or in a dual-chamber system. In another preferred embodiment, the lyophilized or spray-dried bacteria in the first chamber may be suspended in a lipid or oil. This will significantly facilitate packaging and filling. In addition, the surrounding lipid or oil will protect the bacteria from premature rehydration. Preferably, the bacteria are suspended in ethylhexyl cocoate or dicaprylyl carbonate. The weight ratio of the bacteria to the oil or lipid preferably is between 1:1 and 1:2.

In one embodiment, the skin care composition of the present invention is an aqueous preparation, such as a gel. Aqueous preparations as intended herein encompass aqueous solutions, as well as aqueous dispersions. In one embodiment, the skin care composition is an oil-in-water emulsion. If the skin care composition contains an oil phase, e.g. when using an oil-in-water emulsion, it is preferred that the oil phase contains triglycerides and/or octyldodecanol. In addition, the oil phase may contain one or more oils selected from the group of lecithin, olive oil, sunflower oil, jojoba oil, soya oil, peanut oil, rapeseed oil, almond oil, palm oil, coconut oil, castor oil, wheat germ oil, grape seed oil, safflower oil, evening primrose oil, macadamia nut oil and the like.

The term "comprising", when used in the context with methods or compositions, means that other method steps or components of the composition can be present in addition to the method steps or components presented. The use of the term "comprising" indicates inclusion rather than limitation. For example, a composition "comprising" components A+B may also comprise C as a further component. Similarly, a method "comprising" steps (a) and (b) may also comprise (c) as a further method step. In contrast, the term "consisting of", when used in the context with methods or compositions, refers to methods or compositions which are exclusive of any other method steps or components of the composition not recited in the description of the respective composition or method. For example, a composition "consisting of" components A+B is limited to these two components and does not contain any other component apart from A and B. Similarly, a method "consisting of" steps (a) and (b) is a twostep method and does not contain any other method steps apart from (a) and (b). It should be understood, however, that any method or composition described herein as "comprising" certain method steps or components may preferably "consist essentially of" or may more preferably "consist of" the recited method steps or components. Further, unless otherwise required by context, singular terms shall include pluralities and plural terms shall include the singular.

### EXAMPLES

The following examples describe certain preferred embodiments of the present invention. It is however to be noted that the invention is not limited to such embodiments.

### Example 1: Sample acquisition and processing

Swab samples were collected from 30 volunteers (female, n=14; male, n=16) with an age range of 22-43 years from forehead, cheek, back and forearm skin, as described previously (Ahle et al., 2020. In brief, an area of 25 cm² of forehead, cheek, back skin and 50 cm² on forearm skin was swiped with a cotton swap which was pre-moistened in aqueous sampling buffer containing disodium phosphate (12.49 g/L, Merck), potassium dihydrogen phosphate (0.63 g/L, Merck) and 1 % Triton X-100 (Sigma). The swap was vigorously shaken in a tube containing 2 mL of sampling buffer and then removed. The sample was stored at -20°C before DNA extraction. Skin hydration and sebum content were measured with a Corneometer (Courage + Khazaka electronic) and Sebumeter (Courage + Khazaka electronic), respectively. None of the volunteers had a history of skin disease, nor had undergone treatment with topical medicine or antibiotics in the last six months. Written informed consent was obtained from all volunteers and the study was approved by International Medical & Dental Ethics Commission GmbH (IMDEC), Freiburg (Study no. 67885).

The swab samples were diluted (back, cheek, forehead skin sample: 1:10 and 1:1000; forehead skin sample: 1:1 and 1:100) in 0.9 % NaCl solution. Cultivation was done by plating on Columbia agar with 5 % sheep blood; agar plates were incubated at 37°C for 24 h. CFU count was determined with an automatic colony counter (IUL). Up to five colonies that resembled staphylococci based on colony size and color were randomly picked of each plate and pure cultures were obtained by sub-cultivation on the same agar. Each isolate of the 572 isolates that were obtained in total was assigned to species level by MALDI-TOF mass spectrometry.

Prior to DNA extraction, skin swab samples were centrifuged (8.000 g, 30 min at 4°C), and the supernatant was discarded. The pellets were lysed by using lysostaphin (0.05 mg/mL, Sigma) and lysozyme (9.5 mg/mL, Sigma). DNA was extracted using the DNeasy PowerSoil Kit (QIAGEN), following the manufacturer's instructions. DNA concentrations were measured with the Qubit dsDNA HS Assay (ThermoFisher Scientific) using a Qubit fluorometer.

Results: A total of 572 bacterial isolates were obtained via selective cultivation, of which 557 were identified as coagulase-negative staphylococci (CoNS) via MALDI-TOF mass spectrometry. Across all skin sites, the majority of isolates were identified as *S. epidermidis* (n=374, 67.2 %), followed by *Staphylococcus hominis* (n=86, 15.4 %). Forehead, cheek and back skin sites were dominated by strains of *S. epidermidis,* followed by *Staphylococcus capitis* (relative abundance of 74.5% and 11.7 %, respectively), whereas on forearm skin sites, a larger number of strains of *S. hominis* and *Staphylococcus haemolyticus* (relative abundance of 38.7 % and 7.3%, respectively) were isolated. Moisture content (skin hydration) was highest on back and forehead skin as compared to cheek and forearm skin (Fig. 1). The latter sites exhibited the highest sebum content and numbers of staphylococci per cm² (colony forming units/CFU), whereas forearm skin sites were particularly low in sebum and numbers of staphylococci (Fig. 2 and 3).

### Example 2: Effect of S. epidermidis on the abundance of C. acnes on skin

All 557 CoNS isolates were screened for antimicrobial properties against the indicator strain DSM1897 to identify staphylococcal isolates with bioactivity. For this purpose, bacterial lawn plates were prepared for the indicator strains. Liquid cultures of *C*. *acnes* indicator strains were prepared in CASO broth. For *C*. *acnes* strain cultures the liquid culture was adjusted to an optical density of OD₆₀₀ₙₘ =0.075. 6 mL of the adjusted culture was pipetted onto a rectangular Tryptic Soy Agar (TSA) plate and distributed evenly. For round TSA plates 3 mL bacterial suspension was used. After 30 sec excess liquid was removed and the plates were dried for 4 h. The plates were stored up to three weeks at 4°C.

The CoNS isolates were cultivated for 20 h at 37°C shaking in 1 mL CASO broth in 96-Deepwell plates. The 96-Deepwell plate was centrifuged at 2000 rpm for 5 min, 500 µL supernatant was removed and the pellet was resuspended in the remaining liquid. The concentrated bacterial cultures were transferred into 96-well U-bottom plates. With a replicator stamps bacterial cultures were transferred on rectangular lawn plates. After 4 h of drying, the plates were cultivated with varying conditions (*S. aureus* lawn plates: 24, 37°C; *C. acnes* lawn plates: 4-5 days, 37°C, in anaerobic container with AnaeroGen bag (Thermo Scientific)). A visible inhibition zone around a staphylococcal colony was regarded as antimicrobial activity. Staphylococcal strains that showed antimicrobial properties were verified in triplicates. These strains were further tested against eleven different *C*. *acnes* indicator strains from six different SLST classes.

Results: It was observed that a total of 30 of the tested *Staphylococcus* strains showed activity against the *C*. *acnes* strain. These isolates were further screened against eleven different *C*. *acnes* strains covering six different SLST classes (A, C, D, H, K, L), including both acne- and healthy skin-associated types, in order to identify any phylotype-specific bioactivity. Of these 30 staphylococcal isolates, 17 were identified as *S. capitis,* six as *S. hominis,* five as *S*. *epidermidis* and two as *S. warneri.* Strains belonging to different *C*. *acnes* phylogenetic clades showed a remarkably different susceptibility to the antimicrobial activity of the various staphylococcal isolates. The two A-class C. acnes strains, DSM1897 and 12.1.L1, were most susceptible to this bioactivity, being inhibited by the antimicrobial activity and were inhibited by a total of 29 and 15 staphylococcal strains, respectively.

To provide insight into the in vivo relevance of these observations, we compared the relative abundance profiles of *C*. *acnes* populations originating from skin samples with and without the presence of antimicrobial active staphylococcal strains. Notably, the relative abundance of A-class C. acnes was significantly lower in skin sites that contained a staphylococcal strain exhibiting antimicrobial activity. This inverse correlation of abundance was most pronounced in back skin samples; in samples containing antimicrobial-active staphylococci, there was a marked increase in the relative abundance of D-class *C*. *acnes* and a corresponding decrease in A-class *C*. *acnes* respectively (p=0.004 and p=0.0013, respectively).

### Example 3: Preparing a skin care composition

The S. capitis strain HAC349 and the *S. epidermidis* strain HAC588 were incorporated into skin care composition. For this purpose, a freeze-dried powder of *S. capitis* HAC349 and *S*. *epidermidis* HAC588 was produced and mixed with other ingredients as indicated below. Ingredients are listed in % (w/w).

| INCI | % | |
|---|---|---|
| | 1 | 2 |
| Caprylic/Capric Triglyceride | 5 | 5 |
| Glycerin | 10 | 10 |
| Tocopherol | 0.15 | 0.15 |
| Hydrogenated Coco-Glycerides | 4 | 4 |
| Polyglyceryl-3 Diisostearate | 1.5 | 1.5 |
| Decyl Oleate | 17 | 17 |
| Octyldodecanol | 23.8 | 23.8 |
| Hydrogenated Castor Oil | 1 | 1 |
| Helianthus Annuus Seed Oil | 0.15 | 0.15 |
| Cera Alba | 1 | 1 |
| Ricinus Communis Seed Oil | 10 | 10 |
| Butyrospermum Parkii Butter | 8 | 8 |
| Cetyl Palmitate | 4 | 4 |
| Bis-Diglyceryl Polyacyladipate-2 | 4.7 | 4.7 |
| Dicaprylyl Carbonate | 5 | 5 |
| Staphylococcus warneri HAA333 lyophilisate^{∗} | 4.7 | 0 |
| Staphylococcus epidermidis HAC26 lyophilisate^{∗} | 0 | 4.7 |

| | | |
|---|---|---|
| ^{∗} CFU= 10¹⁰/g | | |

### LITERATURE

Ahle CM, et al. Staphylococcus saccharolyticus: An Overlooked Human Skin Colonizer, Microorganisms 2020, 8.
Lomholt HB, Kilian M. Population genetic analysis of Propionibacterium acnes identifies a subpopulation and epidemic clones associated with acne. PLoS One 5, e12277 (2010).
McDowell A, Nagy I, Magyari M, Barnard E, Patrick S. The opportunistic pathogen Propionibacterium acnes: insights into typing, human disease, clonal diversification and CAMP factor evolution. PLoS One 8, e70897 (2013).
Scholz CF, Jensen A, Lomholt HB, Bruggemann H, Kilian M. A novel high-resolution single locus sequence typing scheme for mixed populations of Propionibacterium acnes in vivo. PLoS One 9, e104199 (2014).
Dagnelie MA, et al. Decrease in Diversity of Propionibacterium acnes Phylotypes in Patients with Severe Acne on the Back. Acta Derm Venereol 98, 262-267 (2018).
Lomholt HB, Scholz CFP, Bruggemann H, Tettelin H, Kilian M. A comparative study of Cutibacterium (Propionibacterium) acnes clones from acne patients and healthy controls. Anaerobe 47, 57-63 (2017).
McDowell A, et al. An expanded multilocus sequence typing scheme for propionibacterium acnes: investigation of 'pathogenic', 'commensal' and antibiotic resistant strains. PLoS One 7, e41480 (2012).
McDowell A, et al. A novel multilocus sequence typing scheme for the opportunistic pathogen Propionibacterium acnes and characterization of type I cell surface-associated antigens. Microbiology (Reading) 157, 1990-2003 (2011).
Nakase K, Hayashi N, Akiyama Y, Aoki S, Noguchi N. Antimicrobial susceptibility and phylogenetic analysis of Propionibacterium acnes isolated from acne patients in Japan between 2013 and 2015. J Dermatol 44, 1248-1254 (2017).
Nakase K, et al. Characterization of acne patients carrying clindamycin-resistant Cutibacterium acnes: A Japanese multicenter study. J Dermatol 47, 863-869 (2020).

## Claims

1. Skin care composition for topical administration to the skin, said composition comprising one or more *Staphylococcus* strains that exert a specific antimicrobial activity against pathogenic *Cutibacterium acnes* strains or culture supernatant from such *Staphylococcus* strain.

2. Skin care composition of claim 1, wherein said one or more *Staphylococcus* strain is selected from the group consisting of:
(a) *S. hominis* strain HAA254, deposited at the DSMZ under accession number DSM 34166,
(b) *S. hominis* strain HAA272, deposited at the DSMZ under accession number DSM 34167,
(c) *S. capitis* strain HAB56, deposited at the DSMZ under accession number DSM 34177,
(d) *S. capitis* strain HAB177, deposited at the DSMZ under accession number DSM 34178,
(e) *S. capitis* strain HAB198, deposited at the DSMZ under accession number DSM 34179,
(f) *S. capitis* strain HAB276, deposited at the DSMZ under accession number DSM 34197,
(g) *S. hominis* strain HAC286, deposited at the DSMZ under accession number DSM 34168,
*(h) S. capitis* strain HAC349, deposited at the DSMZ under accession number DSM 34198,
*(i) S. capitis* strain HAC470, deposited at the DSMZ under accession number DSM 34199,
*(j) S. capitis* strain HAC507, deposited at the DSMZ under accession number DSM 34200,
*(k) S. epidermidis* strain HAC588, deposited at the DSMZ under accession number DSM 34158,
*(l) S. epidermidis* strain HAF242, deposited at the DSMZ under accession number DSM 34159,
*(m) S. capitis* strain HAF401, deposited at the DSMZ under accession number DSM 34153,
*(n) S. epidermidis* strain HAF424, deposited at the DSMZ under accession number DSM 34160, and
*(o) S. epidermidis* HAC26, deposited at the DSMZ under accession number DSM 34117.

3. Skin care composition of claim 1 or 2, wherein said *Staphylococcus* strain is present in the composition in lyophilized or spray-dried form.

4. Skin care composition of any of claims 1-3, wherein said *Staphylococcus* strain is present in the composition in an amount of
(a) 10⁴-10¹¹ CFU/ml of the skin care composition, preferably 10⁷-10¹⁰ CFU/ml of the skin care composition, and/or
(b) at least 0.5% (w/v) of the skin care composition.

5. Skin care composition of any of claims 1-4, wherein said composition comprises
(a) *S. hominis* strain HAA254, deposited at the DSMZ under accession number DSM 34166 in an amount of 10⁴-10¹¹ CFU/ml of the skin care composition,
(b) *S. hominis* strain HAA272, deposited at the DSMZ under accession number DSM 34167 in an amount of 10⁴-10¹¹ CFU/ml of the skin care composition,
(c) *S. capitis* strain HAB56, deposited at the DSMZ under accession number DSM 34177 in an amount of 10⁴-10¹¹ CFU/ml of the skin care composition,
(d) *S. capitis* strain HAB177, deposited at the DSMZ under accession number DSM 34178 in an amount of 10⁴-10¹¹ CFU/ml of the skin care composition,
(e) *S. capitis* strain HAB198, deposited at the DSMZ under accession number DSM 34179 in an amount of 10⁴-10¹¹ CFU/ml of the skin care composition,
(f) *S. capitis* strain HAB276, deposited at the DSMZ under accession number DSM 34197 in an amount of 10⁴-10¹¹ CFU/ml of the skin care composition,
(g) *S. hominis* strain HAC286, deposited at the DSMZ under accession number DSM 34168 in an amount of 10⁴-10¹¹ CFU/ml of the skin care composition,
*(h) S. capitis* strain HAC349, deposited at the DSMZ under accession number DSM 34198 in an amount of 10⁴-10¹¹ CFU/ml of the skin care composition,
*(i) S. capitis* strain HAC470, deposited at the DSMZ under accession number DSM 34199 in an amount of 10⁴-10¹¹ CFU/ml of the skin care composition,
*(j) S. capitis* strain HAC507, deposited at the DSMZ under accession number DSM 34200 in an amount of 10⁴-10¹¹ CFU/ml of the skin care composition,
*(k) S. epidermidis* strain HAC588, deposited at the DSMZ under accession number DSM 34158 in an amount of 10⁴-10¹¹ CFU/ml of the skin care composition,
*(l) S. epidermidis* strain HAF242, deposited at the DSMZ under accession number DSM 34159 in an amount of 10⁴-10¹¹ CFU/ml of the skin care composition,
*(m) S. capitis* strain HAF401, deposited at the DSMZ under accession number DSM 34153 in an amount of 10⁴-10¹¹ CFU/ml of the skin care composition,
*(n) S. epidermidis* strain HAF424, deposited at the DSMZ under accession number DSM 34160 in an amount of 10⁴-10¹¹ CFU/ml of the skin care composition, and/or
*(o) S. epidermidis* HAC26, deposited at the DSMZ under accession number DSM 34117 in an amount of 10⁴-10¹¹ CFU/ml of the skin care composition.

6. Skin care composition of claim 5, wherein said composition comprises two or more the *Staphylococcus* strains in approximately equal concentrations.

7. Skin care composition of any of claims 1-6, wherein said composition further comprises one or more excipients.

8. Skin care composition of claim 7, wherein said one or more excipients comprise an emollient, a filler, a thickener, a solubilizer, an antioxidant, a preservative, a pH adjuster, a binder, a buffering agent, a colorant, a humectants, a exfoliating agent, a preservative, a plant extract, an essential oils, or a fragrance.

9. Skin care composition of any of claims 1-8, wherein said composition is in the form of a gel, cream, ointment or lotion.

10. *Staphylococcus* strain selected from the group consisting of:
(a) *S. hominis* strain HAA254, deposited at the DSMZ under accession number DSM 34166,
(b) *S. hominis* strain HAA272, deposited at the DSMZ under accession number DSM 34167,
(c) *S. capitis* strain HAB56, deposited at the DSMZ under accession number DSM 34177,
(d) *S. capitis* strain HAB177, deposited at the DSMZ under accession number DSM 34178,
(e) *S. capitis* strain HAB198, deposited at the DSMZ under accession number DSM 34179,
(f) *S. capitis* strain HAB276, deposited at the DSMZ under accession number DSM 34197,
(g) *S. hominis* strain HAC286, deposited at the DSMZ under accession number DSM 34168,
*(h) S. capitis* strain HAC349, deposited at the DSMZ under accession number DSM 34198,
*(i) S. capitis* strain HAC470, deposited at the DSMZ under accession number DSM 34199,
*(j) S. capitis* strain HAC507, deposited at the DSMZ under accession number DSM 34200,
*(k) S. epidermidis* strain HAC588, deposited at the DSMZ under accession number DSM 34158,
*(l) S. epidermidis* strain HAF242, deposited at the DSMZ under accession number DSM 34159,
*(m) S. capitis* strain HAF401, deposited at the DSMZ under accession number DSM 34153,
*(n) S. epidermidis* strain HAF424, deposited at the DSMZ under accession number DSM 34160, and
*(o) S. epidermidis* HAC26, deposited at the DSMZ under accession number DSM 34117
or culture supernatant obtained from any of strains (a)-(o) recited above.

11. Skin care composition of any of claims 1-9 or bacterial strain or culture supernatant of claim 10 for use in a method of treating acne, oily skin and/or dry skin in a subject.

12. Skin care composition or bacterial strain or culture supernatant for use in a method of claim 11, wherein said composition is for daily administration.

13. Skin care composition of any of claims 1-9 or bacterial strain or culture supernatant of claim 10 for use in a method of reducing the number of pathogenic *C*. *acnes* cells on the skin of a subject.

14. Skin care composition of any of claims 1-9 or bacterial strain or culture supernatant of claim 10 for use in a method of treating an infection of the skin of a subject with pathogenic *C*. *acnes* strains.
